# EUROPEAN PATENT APPLICATION

(11) **EP 4 549 801 A1**
(43) Date of publication of application: **07.05.2025**
(21) Application number: 23207892.3
(22) Date of filing: 06.11.2023
(51) Int. Cl.: F16M 11/08, F16M 11/20, F16M 11/42, F16M 13/02, A61G 12/00, G16H 15/00

(54) **MONITOR SUPPORT**

(71) Applicant: Koninklijke Philips N.V., 5656 AG Eindhoven (NL)
(72) Inventor: DE BOER, Jacob, Eindhoven (NL); VERMA, Mahesh, Eindhoven (NL)
(74) Representative: Philips Intellectual Property & Standards

(57) **Abstract**

A monitor support arrangement including a mounting bar with a pivot, wherein the pivot has a vertical axis around which the mounting bar is able to rotate. The monitor support arrangement also includes two monitor carriers that are each pivotable about the mounting bar, wherein each monitor carrier is configured to receive a monitor with an image screen.

## Description

### FIELD OF THE INVENTION

The present invention relates to a monitor support arrangement.

### BACKGROUND OF THE INVENTION

Dual monitor screens and their respective mounting arrangements are typically secured to face one direction. It is difficult to easily manipulate the positioning of the monitors in other directions because monitor supports are typically rigid and generally immovable.

This is particularly difficult in medical environments, where it may be desirable for an operator or patient to view a monitor at the same time as a surgeon performing an operation. This is difficult because there is little space in an operating theatre, so it is difficult for the surgeon to be on the same side as the operator/patient to view the monitors. It is also difficult to maintain sterility of the operating theatre in this manner as the surgeon moves from the operation field to view the screens. This leads to monitor arrangements that have cumbersome designs that are not easily viewed or transported.

It is a general objective of the present invention to eliminate or at least reduce the above problems.

### SUMMARY OF THE INVENTION

A first aspect of the invention provides a monitor support arrangement comprising: a mounting bar with a pivot, wherein the pivot has a vertical axis around which the mounting bar is able to rotate; two monitor carriers that are each pivotable about the mounting bar, wherein each monitor carrier is configured to receive a monitor with an image screen.

This arrangement allows the monitor carriers to be independently positioned relative to the mounting bar and the other monitor carrier.

Optionally, the monitor carriers are configurable to be positioned in a first viewing position wherein the monitor carriers are arranged so that the monitor image screens are directed in substantially similar directions, a second viewing position wherein the monitor carriers are arranged so that the monitors have their image screens directed in substantially different directions, and a stowed position wherein the monitor carriers are arranged so that the monitors have their image screens directed substantially towards each other; and wherein the monitor carriers are displaceable between the first, second and stowed position.

In the first viewing position, the monitor carriers are positioned in substantially similar directions when the monitor image screens are viewable from one side of the monitor support arrangement. The monitor image screens do not need to be in identical orientations to be positioned in the first viewing position.

In the second viewing position, the monitor carriers are positioned in substantially different directions when the monitor image screens are viewable from different side of the monitor support arrangement. The monitor image screens do not need to be in opposite orientations to be positioned in the second viewing position.

In the stowed position, the monitor carriers are positioned in substantially towards each other so that the monitor image screens are not viewable. The monitor image screens do not need to be directly opposite each other to be positioned in the stowed position.

In the first viewing position, the monitor carriers are viewable from one side of the monitor support arrangement.

In the second viewing position, the monitor carriers are viable from multiple sides of the monitor support arrangement so that multiple people/parties may view the monitor image screens.

In the stowed position, the monitor carriers are positioned in a compact arrangement.

Optionally, each monitor carrier is rotatable around a vertical axis of the mounting bar.

The monitor carrier is arranged to maintain the orientation of the monitor image screen as the monitor carrier is rotated.

Optionally, the monitor support arrangement further comprises at least one carrier arm between each of the monitor carriers and the mounting bar, wherein each carrier arm has a first pivot attached to the monitor carrier, and/or a second pivot attached to the mounting bar so that the monitor carriers are pivotably attached to the mounting bar by the respective carrier arms.

The carrier arms are therefore freely rotatable relative to the mounting bar. The monitor carrier are also freely rotatable relative to the carrier arm. The rotation of the carrier arms enables the monitor carrier to be positioned in any desirable position relative to the mounting bar.

Optionally, the first pivots of the carrier arms are inboard of an outer edge of the monitor carrier.

Optionally, the first pivots of the carrier arms are offset from a center of the monitor carrier.

The monitor carriers may therefore freely rotate without contacting the other monitor carrier as the monitor carriers are positioned in the first, second and stowed positioned.

Optionally, in the stowed position, the edges of the monitor carriers are aligned so that image screens of the monitors are not offset from each other. Such "alignment" means "mostly aligned" taking into account some tolerances acceptable for the use by the user of such monitor support arrangement if used with screens for display purpose.

The monitor support arrangement is compact in the stowed position. The monitor support arrangement therefore occupies less space in the stowed arrangement.

Optionally, the monitor carriers are mounted to the mounting bar so as to effectively have a rotation of freedom of at least 90° around a vertical axis of the mounting bar pivot.

Optionally, the rotation of freedom of the monitor carriers relative to the mounting bar pivot is approximately 180°.

Optionally, the rotation freedom of the monitor carriers relative to the mounting bar pivot is substantially more than 180°.

The monitor carriers are therefore freely positionable relative to the mounting bar. Optionally, a first one of said monitor carriers is displaceable from its first viewing position or second viewing position to its stowed position by rotation around a vertical axis with respect to the mounting bar pivot over approximately 90° in a first direction, and
wherein the second one of said monitor carriers is displaceable from its first or second viewing position to its stowed position by rotation around said vertical axis with respect to the mounting bar pivot over approximately 90° in the opposite direction.

The image screens carried by the monitor carriers are therefore viewable from opposite sides of the monitor support arrangement. This may be advantageous in arrangements where multiple people need to view the image screens carried by the monitor carriers.

Optionally, in the second viewing position, the edges of the monitor carriers are offset so that the image screens of the monitors are offset from each other.

The offset arrangement of the monitor carriers minimizes the distance between the monitor carriers and may be advantageous for viewing the image screens carried by the monitor carriers in space constrained environments.

Optionally, the monitor support arrangement further comprises a third monitor carrier pivotably mounted to the mounting bar, wherein the third monitor carrier is configured to receive a monitor with an image screen.

Optionally, the monitor support arrangement further comprises a carrier arm between the third monitor carrier and the mounting bar, wherein the carrier arm has a first pivot attached to the third monitor carrier and/or a second pivot attached to the mounting bar so that the third monitor carrier is pivotably attached to the mounting bar by the carrier arm.

The carrier arms are freely rotatable relative to the mounting bar. The monitor carrier are also freely rotatable relative to the carrier arm. The rotation of the carrier arms enables the monitor carrier to be positioned in any desirable position relative to the mounting bar.

Optionally, the third monitor carrier pivotably mounted to the mounting bar between the first monitor carrier and the second monitor carrier.

Optionally, the third monitor carrier is configured to attach to a monitor with an image screen, and wherein the third monitor carrier is configurable to be positioned in a first viewing position, wherein the third monitor carrier is arranged so that the monitor image screen is directed in substantially similar directions to the monitor image screens carried by the first and/or second monitor carriers;
a second viewing position, wherein the third monitor carrier is arranged so that the monitor image screen is directed in substantially different directions to the monitor image screens carried by the first and/or second monitor carriers; and
a stowed position, wherein the third monitor carrier is arranged so that the monitor image screen is directed substantially towards one of the monitor image screens carried by the first and second monitor carriers; wherein the third monitor carrier is displaceable between the first, second and stowed position.

Optionally, the monitor support system further comprises a vertical mounting column attached to an apparatus device, wherein the mounting bar is attached to the vertical mounting column so that the monitor carriers are vertically displaceable with respect to the apparatus.

The height of the monitor support system may be changed depending on the application of the monitor support system, such as the position or the height of the people viewing the image screens.

Optionally, the monitor arrangement is for use with an X-ray apparatus, and the monitor carriers are arranged to receive monitors with image screens for displaying X-ray images.

The monitor support arrangement may be viewed during medical procedures. This may be advantageous in the second viewing position, as the monitor support system may be viewed by a patient and doctor simultaneously.

Optionally, the medical apparatus is a mobile carriage, and further comprises control means including at least one human-operable control input device, for controlling the operation of an X-ray apparatus.

Another aspect of the present invention provides a monitor support arrangement comprising a mounting bar with a pivot, wherein the pivot has a vertical axis around which the mounting bar is able to rotate, at least three monitor carriers that are pivotable on the mounting bar, wherein each monitor carrier is configured to attach to a monitor with an image screen, wherein the monitor carriers are configurable to be positioned in a first viewing position, wherein the monitor carriers are arranged so that monitor image screens are directed in the substantially same direction, a second viewing position, wherein the monitor carriers are arranged so that the monitors have their image screens directed in substantially opposite directions; and a stowed position, wherein the monitor carriers are arranged so that the monitors have their image screens directed substantially towards each other; and wherein the monitor carriers are displaceable between the first, second and stowed position.

Optionally, each monitor carrier is rotatable around a vertical axis of the mounting bar.

Optionally, the monitor support arrangement further comprises at least two carrier arms between at least two of the monitor carriers and the mounting bar, wherein each carrier arm has a first pivot attached to the monitor carrier, and a second pivot attached to the mounting bar so that at least two of the monitor carriers are pivotably attached to the mounting bar by the respective carrier arms.

Optionally, the at least three monitor carriers include at least one monitor carrier pivotably attached to the mounting bar.

These and other aspects of the invention will be apparent from and elucidated with reference to the embodiment(s) described hereinafter.

### BRIEF DESCRIPTION OF THE DRAWINGS

Further features and advantages of the present invention will be described below, by way of example only, with reference to the accompanying drawings in which:
FIG. 1 shows an exemplary monitor support arrangement and medical apparatus;
FIG. 2 shows a rear view of exemplary monitors;
FIG. 3A shows a schematic front view of an exemplary monitor support arrangement;
FIG. 3B shows a schematic plan view of an exemplary monitor support arrangement;
FIG. 4 shows an exemplary view of a monitor carrier;
FIGS 5A and 5B show schematic views of an exemplary monitor support arrangement in a first viewing position;
FIGS 6A to 6C show schematic views of an exemplary monitor support arrangement in a second viewing position;
FIGS 7A and 7B show schematic views of an exemplary monitor support arrangement in a stowed position;
FIGS 8A and 8B show further embodiments of the exemplary monitor support arrangement;
FIG. 9 shows another exemplary monitor support arrangement;
FIG. 10 shows a rear view of the exemplary monitor support arrangement;
FIG. 11A shows a schematic front view of an exemplary monitor support arrangement;
FIG. 11B shows a schematic plan view of an exemplary monitor support arrangement;
FIG. 12 shows an exemplary monitor support arrangement in a stowed position;
FIGS 13A and 13B show further embodiments of the exemplary monitor support arrangement.

### DETAILED DESCRIPTION

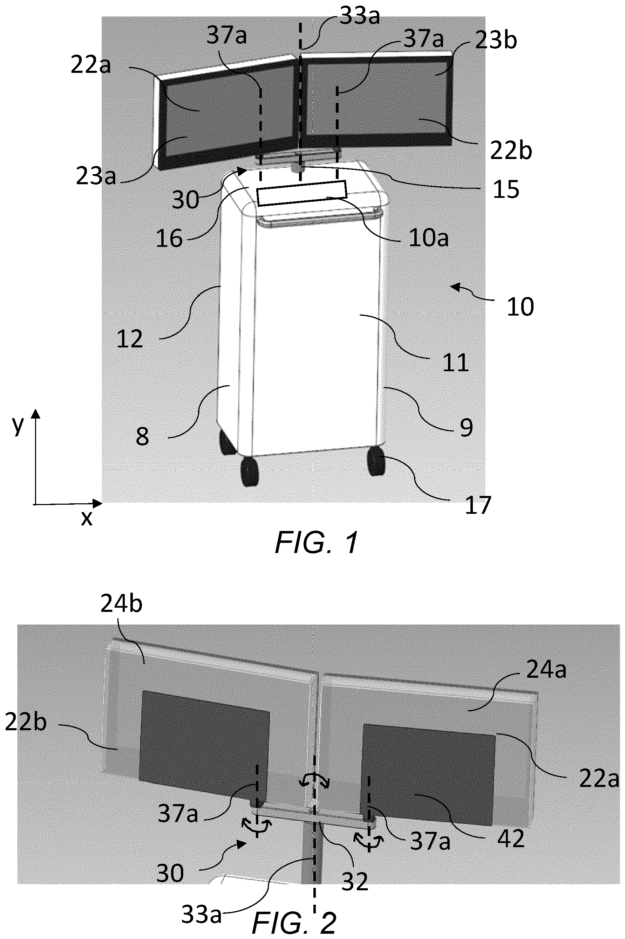

FIGS 1 and 2 show an exemplary mobile carriage 10 for an X-ray examination apparatus, with a front side 11, a back side 12 opposite the front side 11, and two opposite left and right sides 8 and 9. As shown, the carriage 10 has wheels 17 that are used to move the carriage 10. The carriage 10 has a vertical mounting column 15 that extends from an upper surface 16 of the carriage. The carriage 10 also includes control means 10a that includes at least one human-operable control input device, such as a keyboard. The input device may be used for controlling the operation of the X-ray examination apparatus. In this example, the mobile carriage 10 has two monitors 22a and 22b that are mounted to the carriage 10 by a monitor support arrangement 30.

In this example, the carriage 10 has two monitors 22a, 22b. The monitors 22a, 22b are substantially identical and features of each monitor are denoted by 'a' and 'b' respectively. The monitors 22a, 22b are shown in this example to be mounted at the same height, but it will be understood that the monitors 22a, 22b may be positioned at different heights relative to the upper surface 15 of the carriage 10. The monitors 22a, 22b are preferably flat screen monitors, but may be any other suitable monitor arrangement.

Each monitor 22a, 22b has an image screen 23a, 23b, where an image is displayed, and a back side 24a, 24b opposite the image screen. In this example, the monitors 22a, 22b are positioned closer to the back side 12 of the carriage 10 than the front side 11. As will be discussed in further detail below, the image screens 23a, 23b of the monitors 22a, 22b are arranged to be positioned relative to each other using the monitor support arrangement 30. In this example, the image screens 23a, 23b are arranged to display X-ray images.

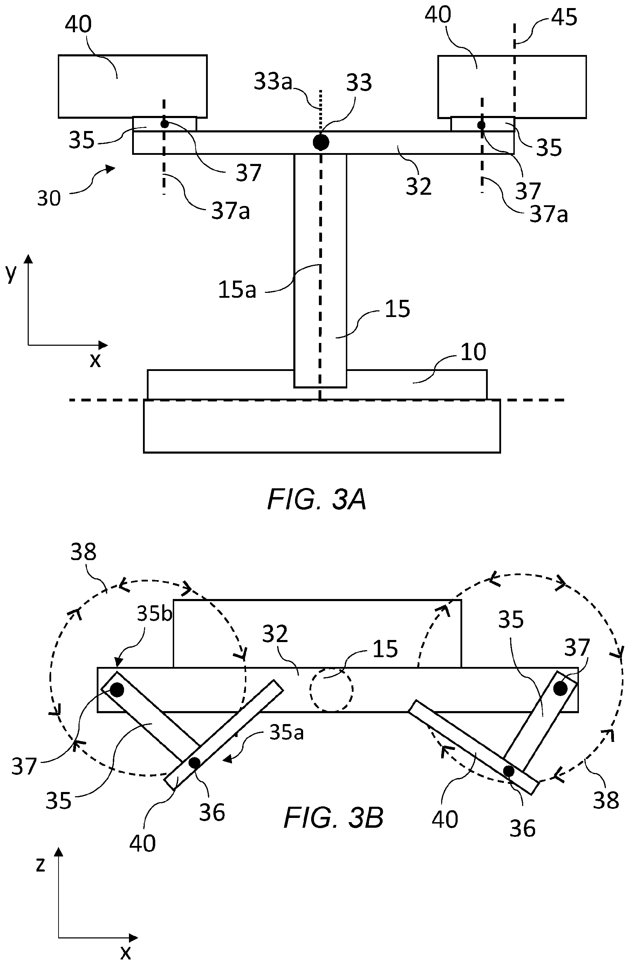

A schematic front view and plan view of an exemplary monitor support arrangement 30 is shown in more detail in FIGS 3A and 3B. As shown, the monitor support arrangement 30 is mounted onto a vertical mounting column 15 that is attached to the carrier 10. The vertical mounting column 15 has a central axis 15a, that runs longitudinally along a length of the mounting column 15. The monitor support arrangement 30 has a mounting bar 32 that is pivotably attached to the vertical mounting column 15 at a pivot 33. In this example, the pivot 33 is located at the midpoint of the mounting bar 32, and the mounting bar 32 is mounted centrally onto the vertical mounting column 15. The pivot 33 in FIG. 3A is therefore equidistant from either end of the mounting bar 32. As shown, the pivot 33 has a vertical axis 33a that is coincident with the vertical axis 15a of the vertical column 15. The mounting bar 32 is able to rotate (in the X-direction) around the vertical axis 33a of the pivot 33. The mounting bar 32 therefore is free to rotate relative to the vertical mounting column 15.

Two carrier arms 35 are pivotable on the mounting bar 32. In this example, the carrier arms 35 are attached to the mounting bar 32 at the distal ends of the mounting bar 32. In other examples, the carrier arms 35 may be positioned inboard of the distal ends of the mounting bar 32. In the example shown in FIGS 3A and 3B, the carrier arms are bars with a first end 35a that is pivotably attached to a monitor carrier 40, and a second end 35b that is pivotably attached to the mounting bar 32. More specifically, the carrier arms 35 have a first pivot 36 attached to the monitor carrier 40, and a second pivot 37 attached to the mounting bar 32. The first end 35a of the carrier arm 35 is attached by the first pivot 36 to the monitor carrier 40, while the second end 35b of the carrier arm 35b is attached by the second pivot 37 to the monitor bar 32. In other examples, the monitor carrier 40 may only have the first pivot 36 or the second pivot 37. Each monitor carrier 40 is pivotable about the mounting bar 32. The first pivot 36 has a vertical axis 36a (in the y-direction) around which the monitor carrier 40 is rotatable around (in the y-direction). Similarly, the second pivot 37 has a vertical axis 37a (in the Y-direction). The carrier arms 35 are rotatable around the vertical axis 37a in relation to the mounting bar 32.

Each monitor carrier 40 is therefore independently rotatable relative to the other monitor carrier 40, carrier arm 35, and the mounting bar 32.

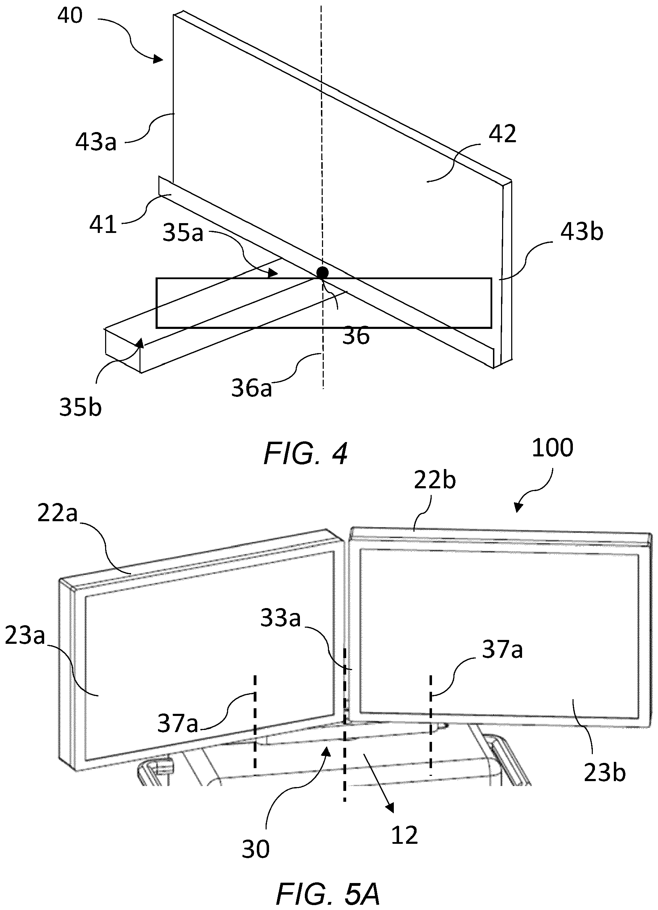

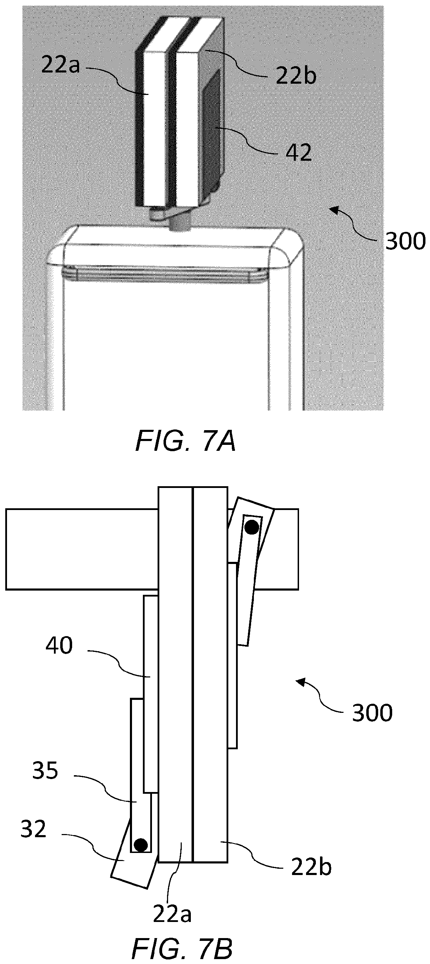

The monitor carrier 40 is shown in greater detail in FIG. 4. As shown, the monitor carrier 40 is mounted by a first pivot 36 to the first end 35a of the carrier arm 35. The monitor carrier 40 includes a supporting back 42 that extends perpendicularly from a base lip 41 that is arranged to receive a monitor 22a, b. As shown more clearly in FIGS 2 and 7A, the supporting back 42 of the monitor carrier 40 extends along a back side 24a, 24b of the monitors 22a, 22b. However, it will be clear to the skilled person that any suitable mounting arrangement may be used for the monitor carrier 40.

As shown in FIG. 3B, each carrier arm 35 is rotatable along respective pathways 38. As shown, the carrier arms 35 are rotatable from 0° to 360° around the mounting bar 32. The diameter of the rotational pathways 38 are customizable depending on the length of the carrier arms 35. Preferably, the length of the carrier arms 35 are such that the carrier arms 35 may freely rotate from 0° to 360° without physically contacting the other carrier arm 35. The carrier arms 35 are therefore rotatable around a vertical axis 37a of the mounting bar 32 unobstructed.

The monitors 22a, 22b are preferably also adjustable in the vertical direction, i.e. the height of the monitors 22a, 22b can be adjusted by moving the vertical column 15 in and out of the carriage 10. Since means for mounting a screen such that its vertical position is adjustable are known per se, a more detailed description of the design of such mounting means is omitted here.

The monitor carriers 40 are mounted to the mounting bars 32 through the carrier bars 35. As the monitor carriers 40 also are rotatable around the first pivot 36, so the monitor carriers 40 effectively have a rotation of freedom of at least 90° around the first pivot 36. Preferably, the rotation of freedom of the monitor carriers 40 relative to the mounting bar pivot 30 is 180°. In other examples, the rotation of freedom of the monitor carriers may be more than 180°. In other examples, the rotation of freedom of the monitor carriers 40 relative to the mounting bar pivot 30 may be less than 180°. The monitor carriers 40 may preferably rotate between 0° and 360° relative to the vertical axis 33a of the mounting bar pivot 33. This enables the monitor carriers 40 to be able to be moved between a first viewing position 100, a second viewing position 200 and a stowed position 300, as discussed in greater detail below.

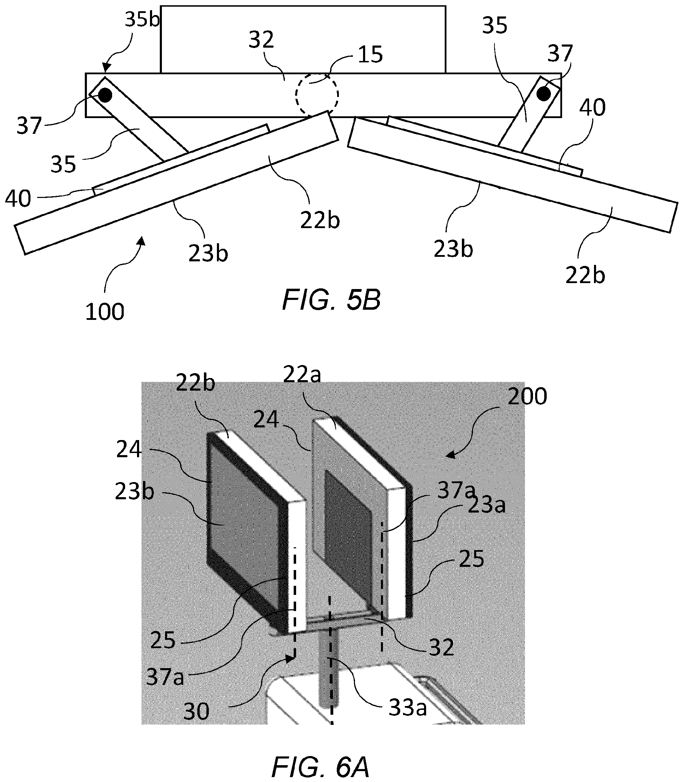

FIGS 5A and 5B show perspective and plan views of the monitor support arrangement 30 and monitors 22a, 22b in the first viewing position 100. As shown more clearly in FIG. 5B, in the first viewing position the monitor carriers 40 are arranged so that the monitor image screens 23a, 23b are directed in substantially similar direction. In this example, the monitor image screens 23a, 23b are directed towards a front side 12 of the carriage 10. In order to be facing in a similar direction, it is noted that the image screens 23a, 23b do not need to be parallel in the same direction. Instead, the majority of the image screens 23a, 23b need to be facing in a similar direction. Preferably, the angle between the two screens is greater than 160 degrees. In other examples, the monitor support arrangement 30 may be in the first viewing position with the image screens 23a, 23b facing towards the rear 11 of the carriage 10.

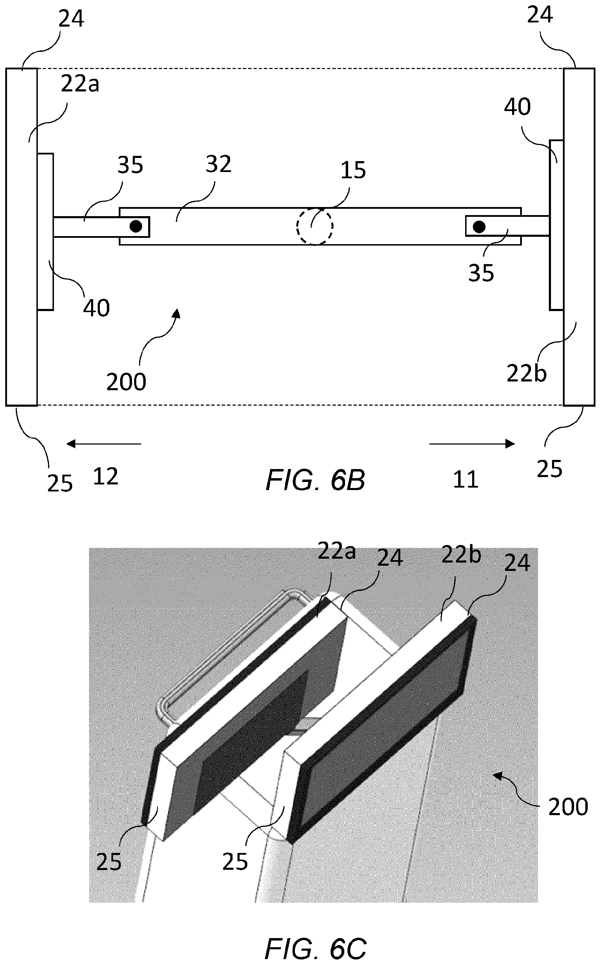

FIGS 6A and 6B show perspective and plan views of the monitor support arrangement 30 and monitors 22a, 22b in the second viewing position 200. As shown more clearly in FIG. 6B, in the second viewing position the monitor carriers 40 are arranged so that the monitor image screens 23a, 23b are directed in substantially different directions. In this example, one monitor image screen 23a is directed towards the front 12 of the carriage 10, while the other monitor screen 22a is directed towards the rear 11 of the carriage 10. However, in order to be facing in different directions, it is noted that the image screens 23a, 23b do not need to be 180° opposite. For example, one image screen 23a may be directed towards a side of the carriage 10 (i.e. at 90° to the mounting bar 32 shown in FIG. 6B), while the other image screen 23b may be directed towards the rear 11 of the carriage 10. In order to be directed in different directions, the majority of the image screens 23a, 23b need to not be facing the same direction.

In the example shown in FIGS 6A and 6B, the respective outer edges 24, 25 of each monitor 22a, 22b are aligned. The monitors 22a, 22b in this example are therefore not offset from each other.

FIG. 6C shows an alternative arrangement of the monitor support arrangement 30 and the monitors 22a, 22b in a second viewing position 200. In this example, the monitor carriers are offset from each other, so that the image screens of the monitors 22a, 22b are offset from each other. As shown, in this example, the outer edges 24, 25 of the monitors 22a, 22b are offset from each other. Offset means that the monitors 22a, 22b and the monitor carriers are not aligned. In the example shown in FIG. 6B, the carrier arms 35 extend perpendicularly from the mounting bar 32. However, in the example shown in FIG. 6C, the carrier arms 35 do not extend perpendicularly from the mounting bar 32. The angle between the carrier arms 35 are reduced so that the monitors 22a, 22b can be positioned offset from each other.

As shown in FIG. 6C, the distance between the monitors 22a, 22b is reduced. In the example shown in FIG. 6C, the second monitor 22b is positioned off the carriage 10 towards a front side 11 of the carriage. As a result, first monitor 22a is positioned closer to front side 11 of the carriage, and closer to the second monitor 22b. In this example, the first monitor 22a is therefore positioned over halfway over the upper surface 16 of the carriage 10. In the second position 200, a larger area of the upper surface 16 is occupied by the monitor 22a.

As the monitor carriers 40 are rotatable relative to the mounting bar 32, it is easy for the monitors 22a, 22b to be rotated between the first and second viewing positions 100, 200. The mounting bar 32 and the monitor carriers 40 allow the monitors 22a, 22b to be positioned as desired. This can be particularly advantageous in areas such as operating theatres or hospital rooms as described below.

The medical team may therefore move from viewing both monitors 22a, 22b in a first viewing position 100, before monitor support arrangement is moved into a second viewing position 200. The second viewing position 200 may be used for displaying and explaining medical procedures to the patient or may be used by a doctor to perform a surgical procedure.

In an operating theatre or hospital environment, it is important to maintain a sterile barrier between the patient and/or doctor and additional team members in the room, such as the nurses or students. By having the monitor support arrangement 30 according to the present invention, it enables the doctors/patient to be present on one side of the carriage 10, while the remaining team members may be on the opposite side of the carriage. This not only maintains the sterile barrier, but also ensures that all members may view the monitors 22a, 22b.

Furthermore, the monitor support arrangement 30 may be positioned into the second position 200 as shown in FIG. 6C. This may be advantageous, for example, if the doctor/team need to pull one of the monitors 22a, 22b closer for view without disrupting the view of the person on the opposite side of the carriage 10. Additionally, in this exemplary second position 200, the monitors 22a, 22b are moved towards a front side 11 of the carriage 10. The resulting unoccupied upper surface 16 may therefore be used as a storage space (for example, notebooks, medical equipment etc.) while the monitor support arrangement is in the second viewing position 200. In other examples, the monitors 22a, 22b may be moved towards the rear side 12 of the carriage 10.

After use, the monitor support arrangement 30 may be positioned into a stowed position 300 and put for example, in a corner of the room or a storage room. As the stowed position 300 is compact, the monitor support arrangement 30 occupies less space than conventional imaging systems or arrangements.

FIGS 7A and 7B show perspective and plan views of the monitor support arrangement 30 and monitors 22a, 22b in the stowed position 300. As shown more clearly in FIG. 7B, in the stowed position the monitor carriers 40 are arranged so that the monitor image screens 23a, 23b are directed substantially towards each other. When in the stowed position 300, the edges of the monitor carriers 43a, 43b are aligned so that the image screens 23a, 23b of the monitors 22a, 22b are not offset from each other in the X-Y direction. This provides a compact arrangement as the monitors 22a, 23a may be positioned above the top surface 16 of the carriage 10 within its perimeter.

The monitor carriers 40 are displaceable from the first viewing position 100 or second viewing position 200 to the stowed position 300 by rotation around the mounting bar 32 pivot's vertical axis 33a. The first monitor carrier 40 may be moved from the first viewing position 100 or second viewing position 200 to the stowed position 300 by rotating around the vertical axis 33a in a first direction by approximately 90°.The second monitor carrier 40 may be moved from the first viewing position 100 or second viewing position 200 to the stowed position 300 by rotating around the vertical axis 33a in a second opposite direction by approximately 90°. The monitor carriers 40 are therefore easily moved from either the first 100 or second viewing position 200 to the stowed position 300.

With this arrangement, the control means 10a can be positioned towards the front side 11 of the carriage 10 while one of the monitors 22a, 22b can be rotated towards the rear side 12 of the carriage. This can be useful if the carriage 10 is used in an operating environment so that one of the monitors and control means 10a can be kept away from the sterile surgical environment. This may help prevent any bodily fluids released from the patient coming into contact with the control means 10a.

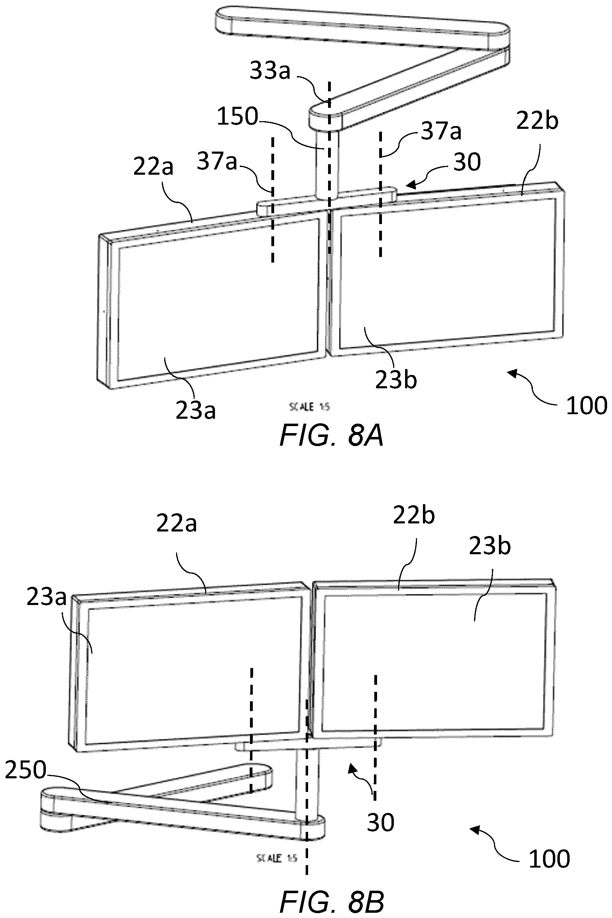

The exemplary monitor support arrangement 30 described in relation to FIGS 1-7B is arranged to be mounted to a vertical mounting column 16 that is received in a medical apparatus 10, such as an X-ray carriage. However, it will be understood that the monitor support arrangement 30 may be used in connection with any number of medical apparatuses, as shown in FIG. 8A.

In this example, the monitor support apparatus 30 is used with a ceiling boom arrangement 150. This arrangement may be beneficial in areas such as operating theatres, where surgeons and trainee doctors or supporting nurses may need to see different image screens 23a, 23b of the monitors 22a, 22b in real time.

In other examples, the monitor support apparatus 30 may be used in non-clinical environments, as shown in FIG. 8B. In this example, the monitor support apparatus 30 is used with a desktop computer support arm 250.

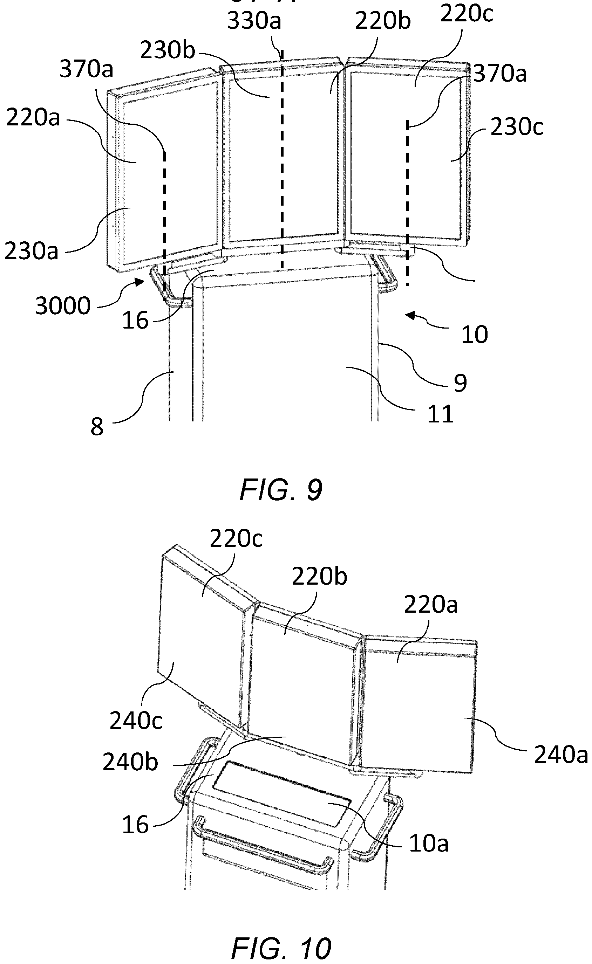

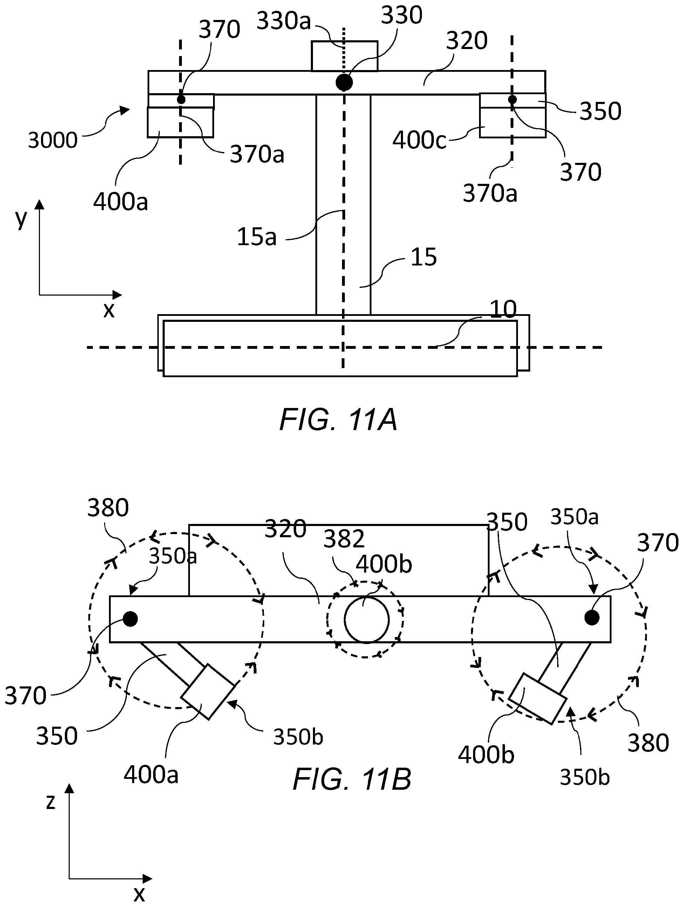

FIGS 9 to 13 show another exemplary embodiment of the monitor support apparatus 3000. As shown, the monitor support apparatus 3000 is also used with a mobile carriage 10 and is mounted to carriage 10 by a vertical mounting column 15 (shown more clearly in FIG. 11A). Components of the carriage 10 and vertical mounting column 15 common to both arrangements have common reference numerals and for brevity will not be described again here.

In this example, the monitor support arrangement 3000 is arranged to carry three substantially identical monitors, 220a, 220b, 220c. The monitors 220a, 220b, 220c are orientated vertically. Similar to the monitors 22a, 22b discussed above, each monitor 220a, 220b, 220c has an image screen 230a, 230b, 230c where an image is displayed, and a back side 240a, 240b, 240c opposite the image screen. In this example, the monitors 220a, 220b, 220c are positioned closer to the front side 11 of the carriage 10 than the back side 12.

A schematic front view and plan view of an exemplary monitor support arrangement 3000 is shown in more detail in FIGS 11A and 11B. The monitor support arrangement 3000 has a mounting bar 320 that is pivotably attached to the vertical mounting column 15 at a pivot 330. The pivot 330 has a vertical axis 330a that is coincident with the vertical axis 15a of the vertical column 15. The mounting bar 320 is therefore able to rotate (in the X-direction) relative to the vertical mounting column 15 around the vertical axis 330a of the pivot 330.

As shown, the central monitor 220b is mounted on to the mounting bar 320 with a monitor carrier 400b. The monitor carrier 400b is arranged to attach to the monitor 220b. In this example, the monitor carrier 400b is a vertical rotatable column (shown more clearly in FIG. 9 in relation to the outermost monitors 220a,c). The monitor carrier 400b may attach directly to a lower side of the monitor 220b, or may be attached to an additional bar or support that receives the monitor 220b (not shown). The monitor carrier 400b has a central axis 460b and is mounted to the mounting bar 320 so that the monitor carrier 400b can rotate around the central axis 360a. In this example, the monitor carrier's central axis 460b is coincident with the vertical axis 15a of the vertical column 15. The monitor carrier 400b is able to rotate around the vertical axis 460b of the monitor carrier 400. In other examples, the central monitor 220b may be attached through a carrier arm or may be directly attached to the mounting bar 320.

Two carrier arms 350 are attached to the mounting bar 320 in a similar manner as described above and will not be repeated for brevity. Components of the carrier bars 350 common to both arrangements have common reference numerals with an addition '0' in reference to the monitor support arrangement 3000, and for brevity will not be described again here.

The first end 350a of the carrier arm 350 is attached by the first pivot 370 to the mounting bar 320, while the second end 350b of the carrier arm 350b is attached to the monitor carriers 400a,c. The first pivot 370 has a vertical axis 370a (in the y-direction) around which the carrier arms 350 is rotatable around the mounting bar 320 (in the y-direction). Each monitor carrier 400a,c is a rotatable column that has a vertical axis 460a,c around which the monitor carrier 400 is rotatable around. Each monitor carrier 400a,b,c is therefore independently rotatable relative to the other monitor carrier 400a,b,c, carrier arm 350, and the mounting bar 320.

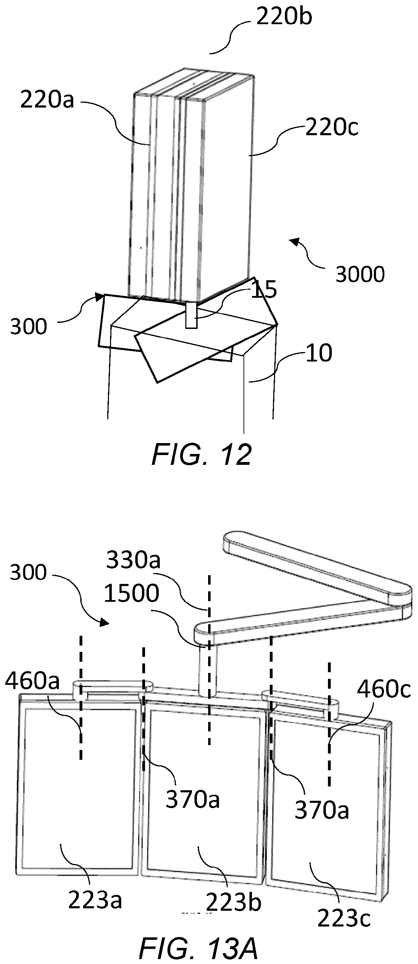

As shown in FIG. 11B, each carrier arm 350 is rotatable along respective pathways 380, while the central monitor carrier 400b is rotatable along the pathway 382. It will be understood that the monitor arrangement 3000 operates in substantially identical way to the monitor arrangement 30 and is therefore positionable between a first viewing position 100, a second viewing position 200 and a stowed position 300. FIG. 12 shows the monitor arrangement in greater detail in the stowed position 300, but it will be understood that the monitor carriers 400a,b,c may be rotatable so that the monitors 220a,b,c can be moved between the first, second and stowed positions 100,200,300.

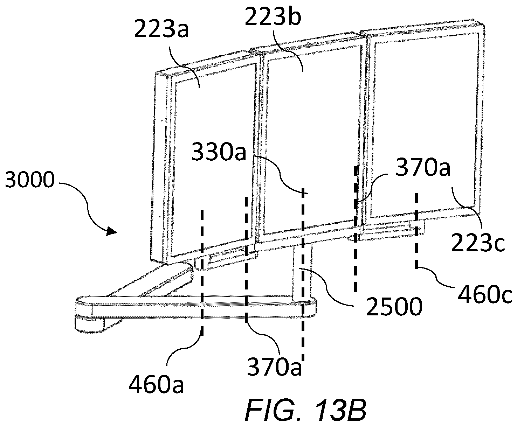

While the monitor support arrangement 3000 is described in relation to a carriage 10, it will be understood that similarly to the monitor support arrangement 30, the monitor support arrangement 3000 may be used in connection with a number of different medical apparatuses. For example, as shown in FIG. 13A, the monitor support apparatus 3000 may be used with a ceiling boom arrangement 1500. In other examples, the monitor support apparatus 3000 may be used in non-clinical environments, as shown in FIG. 13B. In this example, the monitor support apparatus 3000 is used with a desktop computer support arm 2500.

Where the word 'or' appears this is to be construed to mean 'and/or' such that items referred to are not necessarily mutually exclusive and may be used in any appropriate combination.

Although the invention has been described above with reference to one or more preferred embodiments, it will be appreciated that various changes or modifications may be made without departing from the scope of the invention as defined in the appended claims.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A monitor support arrangement comprising:
a mounting bar with a pivot, wherein the pivot has a vertical axis around which the mounting bar is able to rotate;
two monitor carriers that are each pivotable about the mounting bar, wherein each monitor carrier is configured to receive a monitor with an image screen.

2. A monitor support arrangement according to claim 1, wherein the monitor carriers are configurable to be positioned in:
a first viewing position, wherein the monitor carriers are arranged so that monitor image screens are directed in substantially similar directions;
a second viewing position, wherein the monitor carriers are arranged so that the monitors have their image screens directed in substantially different directions; and
a stowed position, wherein the monitor carriers are arranged so that the monitors have their image screens directed substantially towards each other; and wherein the monitor carriers are displaceable between the first, second and stowed position.

3. A monitor support arrangement according to claim 1 or 2, wherein each monitor carrier is rotatable around a vertical axis of the mounting bar.

4. A monitor support arrangement according to any preceding claim, wherein the monitor support arrangement further comprises at least one carrier arm between each of the monitor carriers and the mounting bar, wherein each carrier arm has a first pivot attached to the monitor carrier, and/or a second pivot attached to the mounting bar so that the monitor carriers are pivotably attached to the mounting bar by the respective carrier arms.

5. A monitor support arrangement according to claim 4, wherein the first pivots of the carrier arms are inboard of an outer edge of the monitor carrier.

6. A monitor support arrangement according to claim 5, wherein the first pivots of the carrier arms are offset from a center of the monitor carrier.

7. A monitor support arrangement according to any preceding claim, wherein in the stowed position, the edges of monitor carriers are aligned so that the image screens of the monitors are not offset from each other.

8. A monitor support arrangement according to any preceding claim, wherein a first one of said monitor carriers is displaceable from its first viewing position or second viewing position to its stowed position by rotation around a vertical axis with respect to the mounting bar pivot over approximately 90° in a first direction, and
wherein the second one of said monitor carriers is displaceable from its first or second viewing position to its stowed position by rotation around said vertical axis with respect to the mounting bar pivot over approximately 90° in the opposite direction.

9. A monitor support arrangement according to any preceding claim, wherein in the second viewing position, the edges of monitor carriers are offset, so that the image screens of the monitors are offset from each other.

10. A monitor support arrangement according to any preceding claim, further comprising a third monitor carrier pivotably mounted to the mounting bar, wherein the third monitor carrier configured to receive a monitor with an image screen.

11. A monitor support arrangement according to claim 10, wherein the monitor support arrangement further comprises a carrier arm between the third monitor carrier and the mounting bar, wherein the carrier arm has a first pivot attached to the third monitor carrier and/or a second pivot attached to the mounting bar so that the third monitor carrier is pivotably attached to the mounting bar by the carrier arm, wherein the third monitor carrier is optionally pivotably mounted to the mounting bar between the first monitor carrier and the second monitor carrier.

12. A monitor support arrangement according to claim 11, wherein the third monitor carrier is configured to attach to a monitor with an image screen,
and wherein the third monitor carrier is configurable to be positioned in
a first viewing position, wherein the third monitor carrier is arranged so that the monitor image screen is directed in substantially similar directions to the monitor image screens carried by the first and/or second monitor carriers;
a second viewing position, wherein the third monitor carrier is arranged so that the monitor image screen is directed in substantially different directions to the monitor image screens carried by the first and/or second monitor carriers; and
a stowed position, wherein the third monitor carrier is arranged so that the monitor image screen is directed substantially towards one of the monitor image screens carried by the first and second monitor carriers;
wherein the third monitor carrier is displaceable between the first, second and stowed position.

13. A monitor support arrangement according to any preceding claim, further comprising a vertical mounting column attached to an apparatus, wherein the mounting bar is attached to the vertical mounting column so that the monitor carriers are vertically displaceable with respect to the apparatus.

14. A monitor support arrangement according to claim 13, wherein the monitor arrangement is for use with an X-ray apparatus, and the monitor carriers are arranged to receive monitors with image screens for displaying X-ray images, wherein optionally the medical apparatus is a mobile carriage, and further comprises optionally control means including at least one human-operable control input device, for controlling the operation of an X-ray apparatus.

15. A monitor support arrangement comprising:
a mounting bar with a pivot, wherein the pivot has a vertical axis around which the mounting bar is able to rotate;
at least three monitor carriers that are pivotably attached to the mounting bar, wherein each monitor carrier is configured to attach to a monitor with an image screen;
wherein the monitor carriers are configurable to be positioned in a first viewing position, wherein the monitor carriers are arranged so that monitor image screens are directed in the substantially same direction;
a second viewing position, wherein the monitor carriers are arranged so that the monitors have their image screens directed in substantially opposite directions; and
a stowed position, wherein the monitor carriers are arranged so that the monitors have their image screens directed substantially towards each other; and wherein the monitor carriers are displaceable between the first, second and stowed position.
